# EUROPEAN PATENT APPLICATION

(11) **EP 3 680 620 A1**
(43) Date of publication of application: **15.07.2020**
(21) Application number: 19151205.2
(22) Date of filing: 10.01.2019
(51) Int. Cl.: G01C 21/36, G06F 3/01, G06T 11/00, G02B 27/01, A61M 21/00

(54) **VEHICLE IMAGE DISPLAYING APPARATUS AND METHOD OF DISPLAYING IMAGE DATA ON A DISPLAY DEVICE DISPOSED IN A VEHICLE**

(71) Applicant: Alpine Electronics, Inc., Tokyo 145-8501 (JP)
(72) Inventor: Kizilbagli, Cengiz, 70567 Stuttgart (DE)
(74) Representative: Schmitt-Nilson Schraud Waibel Wohlfrom Patentanwälte Partnerschaft mbB

(57) **Abstract**

A vehicle image displaying apparatus (10) comprises a display device (14) mounted in a vehicle (1) and configured to display one or more images, and a processing device (13) comprising a processor (16) and adapted to be coupled with the display device (14), and configured to receive and display at least one first image (IM1) on the display device (14). At least one of the processing device (13) and the display device (14) is configured to provide a frame-like area (22) simultaneously with displaying the at least one first image (IM1) on the display device (14) such that the at least one first image (IM1) is framed by the frame-like area (22) surrounding the at least one first image (IM1), wherein a position of at least a portion of the frame-like area (22) is controllably movable by the processing device (13). The processing device (13) is configured to determine at least one parameter which is indicative of at least one acceleration (AD1, AD2, AD3) of at least a part of the vehicle (1) during motion of the vehicle, and configured to move during motion of the vehicle (1) the at least one portion of the frame-like area (22) according to the at least one parameter.

## Description

The present invention relates to a vehicle image displaying apparatus, a method of displaying image data on a display device disposed in a vehicle, and a computer program product comprising software code sections to perform such method.

Existing vehicle image displaying systems available for use in vehicles, such as display systems mounted on a dashboard of the vehicle or in a rear seat entertainment system, may provide individual images or image streams (such as a video entertainment program) to occupants of the vehicle via such display devices. For example, a front seat passenger may watch a video stream displayed on the central display of the head unit, while a rear seat passenger watches through a rear seat entertainment system a video on a display device at the rear side of the front seat and listens to the corresponding audio via headphones.

Such known display system setups may have the drawback that vehicle passengers may suffer from motion sickness when they are reading or watching images or image streams, such as video streams, while they are in a moving vehicle. The human body senses acceleration while the vehicle is moving, but the passenger's brain cannot combine this acceleration with the movement of the focused information medium. The passenger is focusing on the information medium, such as a fixedly mounted display device in a car, while the environment of the display device moves. If, for example, a passenger keeps his or her eyes on the information medium, and watches the images or video stream in the moving vehicle, he or she is liable to get motion sickness by reason that visual information is different from the passenger's body's sensory information.

In US 2007/0198183 A1, an image display apparatus is provided which can reduce a burden to be imposed on vehicle occupants by informing the vehicle occupants about current and next travel states of a vehicle while allowing the vehicle occupants to watch TV or the like in the vehicle. In the on-vehicle image display apparatus, the image controlling means controls the on-screen position of the image produced by the image producing means on the basis of the judgments made by travel state detecting means on whether or not the vehicle is turning to the right, or about to turn to the right, whether or not the vehicle is turning to the left, or about to turn to the left, whether or not the vehicle is being accelerated, or about to be accelerated, and whether or not the vehicle is being decelerated, or about to be decelerated. As an example, the image controlling means shifts, in a leftward direction, the on-screen position of the vehicle if the judgment is made that the vehicle is turning to the left, and to shift, in a rightward direction, the on-screen position of the vehicle if the judgment is made that the vehicle is turning to the right.

In US 2008/0062008 A1 there is disclosed an alarm device which comprises a motion detector for detecting a motion of a vehicle, a display located in the vehicle, and adapted to display an image on a screen, and a controller for controlling, on the basis of the motion of the vehicle detected by the motion detector, a display mode in which the image is displayed on the screen by the display. For example, a controlling means changes, on the basis of the degrees of the left-turning, right-turning, accelerating, and decelerating motions of the vehicle, the shape of the image to be displayed on the screen.

Both approaches addressing the problem of motion sickness have a drawback in that images or image streams to be displayed on the display device may become alienated or turbulent as a result of the measures taken to reduce motion sickness, which may reduce the performance of the infotainment system and passengers' experience while the vehicle is in motion.

It is thus an object of the present invention to provide a vehicle image displaying apparatus and a method of displaying image data to a user seated in a vehicle which can keep the performance of the infotainment system unchanged and enhancing the passenger's entertainment experience while reducing motion sickness during motion of the vehicle.

The invention relates to a vehicle image displaying apparatus and a method of displaying image data on a display device disposed in a vehicle according to the appended claims. The invention further relates to a computer program product comprising software code sections which are adapted to perform such method.

In a first aspect, there is disclosed a method of displaying image data on a display device disposed in a vehicle, comprising receiving, by a processing device comprising a processor and adapted to be coupled with the display device, at least one first image for displaying on the display device, displaying, by the processing device, the at least one first image on the display device, and providing a frame-like area simultaneously with displaying the at least one first image such that the at least one first image is framed by the frame-like area surrounding the at least one first image, wherein a position of at least a portion of the frame-like area is controllably movable by the processing device. The processing device determines at least one parameter which is indicative of at least one acceleration of at least a part of the vehicle during motion of the vehicle. The method further includes the step of moving, by the processing device, during motion of the vehicle the at least one portion of the frame-like area according to the at least one parameter.

According to another aspect, there is disclosed a vehicle image displaying apparatus, comprising a display device mounted in a vehicle and configured to display one or more images, and a processing device comprising a processor and adapted to be coupled with the display device, and configured to receive and display at least one first image on the display device. At least one of the processing device and the display device is configured to provide a frame-like area simultaneously with displaying the at least one first image on the display device such that the at least one first image is framed by the frame-like area surrounding the at least one first image, wherein a position of at least a portion of the frame-like area is controllably movable by the processing device. The processing device is configured to determine at least one parameter which is indicative of at least one acceleration of at least a part of the vehicle during motion of the vehicle, and is configured to move during motion of the vehicle the at least one portion of the frame-like area according to the at least one parameter.

The invention thus provides a vehicle image displaying apparatus and a method of displaying image data to a user seated in a vehicle which are capable to enhance the passenger's entertainment experience while reducing motion sickness during motion of the vehicle. While the display of the at least one first image on the display device may stay unchanged, the frame-like area, for example, may display a second image (such as a structured infill picture) which is moving in reaction to measured acceleration, while the display device and the at least one first image stay fix with respect to the car environment, e.g. dashboard. When the passenger is focusing on the displayed first image(s) on the display device, the immediate circumference or surrounding (i.e. the frame-like area) may be moved accordingly so as to match with the sensed acceleration, which corresponds to the acceleration sensed by the passenger's body, thus reducing or preventing motion sickness. Particularly, motion sickness may be reduced or prevented by reason that visual information is no longer different from the passenger's body's sensory information received during vehicle motion.

According to an embodiment, the processing device is configured to display at least one second image simultaneously with the at least one first image on the display device such that the at least one first image is framed by the at least one second image in the frame-like area, and the processing device is configured to move at least one portion of the at least one second image. Advantageously, the display device may comprise two image areas, namely the one displaying the at least one first image and the one displaying the at least one second image which surrounds or frames the at least one first image and moves in accordance with the vehicle's acceleration.

According to another embodiment, the display device comprises at least one frame comprising the frame-like area, wherein the at least one frame is arranged around a display area of the display device for displaying the at least one first image such that the at least one first image is framed by the frame-like area.

According to an embodiment, the at least one frame may comprise one or more light emitting elements, such as LEDs (light emitting diodes), arranged in the at least one portion of the frame-like area.

In a preferred embodiment, the at least one parameter is determined from one or more sensor data provided by at least one acceleration sensor device. Such one or more acceleration sensor devices may be positioned in the vehicle chassis and may also be used for other purposes, such as for controlling the vehicle's driving behaviour in certain driving situations, e.g. sharp braking or driving through sharp curves.

According to an embodiment, the at least one parameter is indicative of at least one acceleration direction of the at least one part of the vehicle. The at least one portion of the frame-like area is moved at least in one or more directions parallel to the at least one acceleration direction. This provides the advantage that the passenger may sense the same acceleration direction as the passenger's body experiences through perception of the moving frame-like area.

According to a further embodiment, the at least one parameter is indicative of at least one first acceleration magnitude of the at least one part of the vehicle, and the at least one portion of the frame-like area is moved with at least one second acceleration magnitude corresponding to the at least one first acceleration magnitude. This provides the advantage that the passenger may sense the same acceleration magnitude as the passenger's body experiences through perception of the moving frame-like area.

According to an embodiment, the at least one parameter is indicative of at least one of a vertical acceleration and a lateral acceleration of the at least one part of the vehicle. These are typically accelerations causing motion sickness.

According to a further embodiment, the at least one parameter is indicative of at least one of a vertical acceleration, a lateral acceleration, and a longitudinal acceleration of the at least one part of the vehicle. In this way, motion sickness may counteracted also taking account of the longitudinal direction, e.g. when sharp braking the vehicle or highly accelerating the vehicle to higher speeds.

According to an embodiment, the method further comprises receiving, by the processing device, at least one second image for displaying on the display device, wherein providing the frame-like area comprises displaying the at least one second image simultaneously with the at least one first image on the display device such that the at least one first image is framed by the at least one second image in the frame-like area, and moving the at least one portion of the frame-like area comprises moving at least one portion of the at least one second image. For example, the at least one second image may be an infill image serving as a background image for the at least one first image and which is moved according to the measured acceleration.

According to an embodiment, the at least one parameter is indicative of at least one of a vertical acceleration and a lateral acceleration of the at least one part of the vehicle. The at least one second image may be a two-dimensional image which is moved in at least one of a vertical and lateral direction of the vehicle corresponding to at least one of the vertical acceleration and lateral acceleration.

According to a further embodiment, the at least one parameter is indicative of at least one of a vertical acceleration, a lateral acceleration, and a longitudinal acceleration of the at least one part of the vehicle. The at least one second image may be a three-dimensional image which is moved in at least one of a vertical, a lateral, and a longitudinal direction of the vehicle corresponding to at least one of the vertical acceleration, lateral acceleration, and longitudinal acceleration.

Preferably, at least one of a dimension, a geometry, and a movement sensitivity (e.g. a magnitude of the acceleration of the movement) of the frame-like area is adjustable through input into a human machine interface by a user. This way, the frame-like area can be adjusted according to the user's needs, which may be different in regards of sensitivity to motion sickness and preferences regarding magnitude of the display area displaying the at least one first image, e.g. a video stream. With the adjustment, the user can adjust the frame-like area to get a good compromise between reducing or suppressing motion sickness and gaining an enlarged user experience.

According to another aspect, the invention also relates to a computer program product comprising software code sections which are adapted to perform a method as set out herein when loaded into an internal memory of the processing device. In particular, such computer program product may be implemented, in principle, in any processing device on the vehicle or outside the vehicle which is appropriate to perform the respective function. Such processing device may be, for example, implemented in, or part of, a processor of an ECU (electronic control unit) of the vehicle, or may be a separate component (e.g. coupled to the CAN-Bus of the vehicle through wire or wirelessly), or may be a distributed system using one or more processors. The computer program may be stored on a computer readable medium, e.g. on the vehicle.

Any aspects and embodiments described herein with respect to the method can equally or analogously be employed in the vehicle image displaying apparatus as described herein, and vice versa, with the processing device and/or display device configured (by software and/or hardware) appropriately.

According to an embodiment, the processing device is comprised in at least one of the following: the vehicle chassis, the display device, and/or a navigation system located in the vehicle. Such processing device may be, for example, implemented in, or part of, a processor of an ECU or a HU (head unit) of the vehicle, or may be a separate component (e.g. coupled to the Ethernet-Bus or CAN-Bus of the vehicle), or may be a distributed system using one or more processors and integrated circuits in or outside the vehicle. For example, the determining of the at least one parameter, such as acceleration, may be performed in a processor of the vehicle's ECU, whereas the providing of the images to the display device and moving the frame-like area ("virtual frame") may be performed by the processor of the display device or HU. The functions performed by the processing device may be implemented in hardware and/or in software.

The present invention can be used, in principle, in any type of vehicle, preferably in a car (automobile) of common type which typically is exposed to a large range of vehicle dynamics during driving. The vehicle image displaying apparatus may be used as a "front seat infotainment system" or a "passenger seat entertainment system" in a manner like a rear seat entertainment system of a known implementation.

Aspects of the invention and embodiments will now be described with reference to the following Figures, in which
- Fig. 1: depicts a schematic view of a vehicle including a vehicle image displaying apparatus according to an embodiment of the invention,
- Fig. 2: is a schematic block diagram of a vehicle image displaying apparatus according to an embodiment of the invention,
- Fig. 3: depicts a schematic view of a display device mounted on or at a dashboard of a vehicle according to an embodiment of the invention,
- Fig. 4A: shows a schematic depiction of a display provided by a display device of a vehicle image displaying apparatus according to an embodiment of the invention,
- Fig. 4B: shows a schematic depiction of a display provided by a display device of a vehicle image displaying apparatus according to another embodiment of the invention.

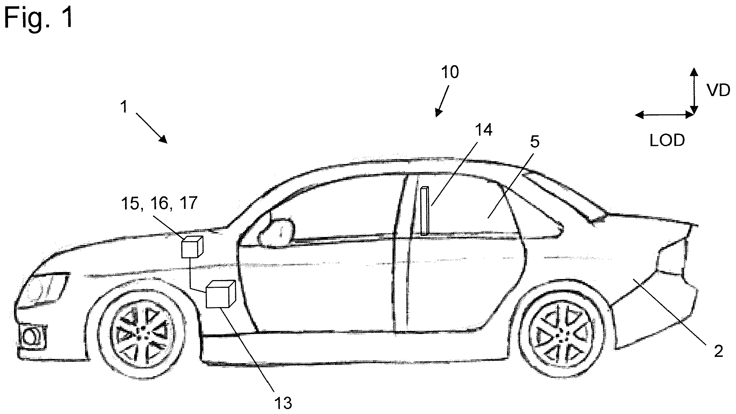

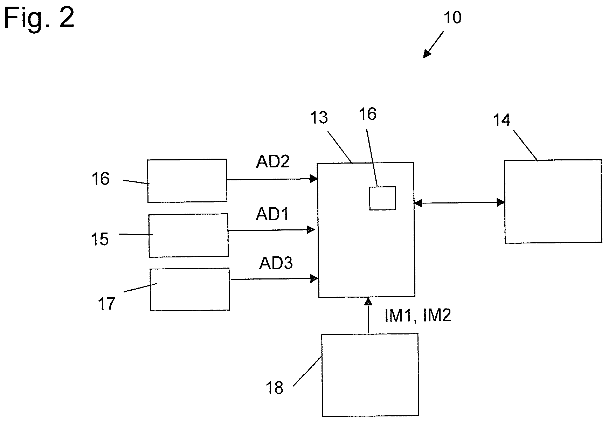

Fig. 1 in combination with Fig. 2 shows a vehicle 1 and a schematic block diagram of a vehicle image displaying apparatus 10 according to an embodiment of the invention, with a schematic view of the vehicle 1 including such vehicle image displaying apparatus 10 according to an embodiment of the invention.

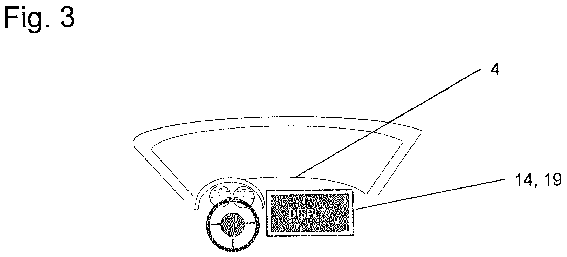

The vehicle 1 generally comprises a vehicle chassis 2 as is known with common vehicles, such as commonly known cars. In a passenger space 5, a driver and one or more passengers (also designated herein as "user" or "passenger"), such as a front seat and/or rear seat passenger travelling in the vehicle, may be seated in a front or back seat of the vehicle 1. In the present embodiment, the vehicle 1 comprises at least one display device 14 which is part of a rear seat entertainment system as commonly known. However, according to other embodiments, the display device 14 may be part of a central head unit or navigation system placed, e.g., on or at a dashboard of the vehicle (see, e.g., Fig. 3).

The vehicle image displaying apparatus 10 basically comprises a processing device 13 which is capable of and configured to be coupled with the display device 14. In use configuration, the processing device 13, which may comprise one or more microprocessors 16 as commonly used in, e.g., vehicle ECUs, or HUs, is coupled with the display device 14, e.g., in wired manner or wirelessly. Other suitable wired and/or wireless configurations and connections between these components are also possible. For example, in a distributed system, the processing device 13 may also comprise or be connected with a so-called Cloud-Server, which is a remote server computer somewhere outside the vehicle, for example coupled through a mobile communications network with the ECU and/or HU of the vehicle, respectively, and to which certain processing or calculation procedures can be outsourced, or from which images, such as video streams, may be received. Therefore, according to embodiments of the invention, the vehicle image displaying apparatus 10 may be a fully or partially vehicle integrated device.

In the present embodiment, the vehicle 1 further comprises at least one acceleration sensor device providing sensor data indicative of at least one acceleration, such as a vertical acceleration, lateral acceleration and/or longitudinal acceleration, of at least a part of the vehicle 1 during motion of the vehicle. According to an embodiment, the at least one sensor device is or comprises one or more acceleration sensors providing acceleration data AD for determining at least one parameter which is indicative of acceleration of the vehicle 1. Generally, any sensor device can be used which provides acceleration data for determining at least one parameter which is indicative of at least one acceleration of an entity which is caused by motion of the vehicle 1. For example, if an acceleration sensor installed in the vehicle is used which is positioned somewhere in the vehicle 1, the measured acceleration is caused by motion of the vehicle. Likewise, an acceleration sensor can be disposed in the display device 14, or elsewhere in an entity which is moved by the vehicle. According to the present embodiment, the vehicle 1 comprises a plurality of sensor devices, i.e. an acceleration sensor 15 for measuring vertical acceleration, an acceleration sensor 16 for measuring lateral acceleration, and an acceleration sensor 17 for measuring longitudinal acceleration of at least one part of the vehicle 1. The acceleration sensors 15, 16, 17 are coupled with the processing device 13 and provide sensor data AD1 indicative of vertical acceleration, AD2 indicative of lateral acceleration, and AD3 indicative of longitudinal acceleration. These sensor data AD1 to AD3 are provided to the processing device 13, as shown in Fig. 2.

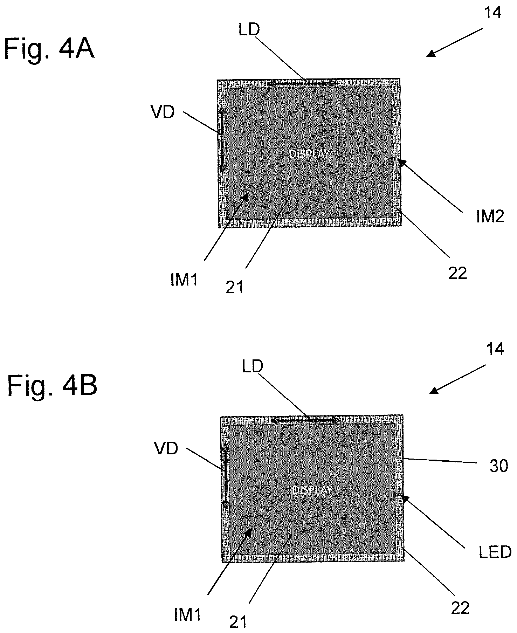

From these sensor data, the processing device 13 determines at least one parameter which is indicative of at least one acceleration, particularly vertical acceleration, lateral acceleration, and/or longitudinal acceleration of at least a part of the vehicle 1 during motion of the vehicle. The determined at least one parameter is indicative of at least one acceleration direction, such as vertical direction VD, lateral direction LD and/or longitudinal direction LOD (Fig. 1 and 4) of the vehicle 1. The determined at least one parameter may also be indicative of an acceleration magnitude of the respective acceleration.

The processing device 13 is further coupled with a storing device 18, which may be a volatile or non-volatile memory for storing any kind of data, such as individual images, movies, or any other video streams. For example, the processing device 13 receives from the storing device 18 a video stream, which is a sequence of first images IM1 to be displayed on the display device 14. For example, as with common rear seat entertainment systems, if the passenger of the vehicle wants to see a movie during the journey, such movie may be provided from the storing device 18 to the processing device 13 as commonly known. For example, the storing device 18 is a memory of a smartphone or tablet computer, or may be a hard drive of the vehicle. The storing device 18 is coupled with the processing device 13 in wired manner or wirelessly, e.g. using common wireless standards.

As shown in Fig. 1, the display device 14 may be positioned in a rear seat environment of the vehicle, or may be positioned on or at a dashboard 4 of the vehicle 1, as shown on Fig. 3, e.g. in a HU of the vehicle. The display device 14 may also be configured as, or part of, a human machine interface 19 which may receive inputs by a user.

In the following, an operation of the vehicle image displaying apparatus 10 according to an embodiment will be described referring to the exemplary depictions of Figs. 4A and 4B:
According to the embodiment of Fig. 4A, the processing device 13 receives an image stream IM1 (i.e., plurality of first images), e.g. a movie, for displaying on the display device 14 which displays the image stream IM1 within an image area 21. The processing device 13 further receives at least one second image IM2, e.g. one or more background images like an infill image, for displaying on the display device 14. The at least one second image IM2 is displayed simultaneously with the image stream IM1 on the display device 14 in an image area 22 which has the form of a frame-like area, such that the image stream IM1 is framed by the at least one second image IM2 in the frame-like area 22.

A position of at least a portion of the frame-like area 22 is controllably movable by the processing device 13. In the embodiment of Fig. 4A, at least a portion of the second image IM2 may be moved by the processing device within the frame-like area 22, and displayed in the frame-like area 22 accordingly. For example, the image IM2 may be moved vertically in vertical direction VD and/or laterally in lateral or transverse direction LD, while the image stream IM1 stays fixed in position within image area 21, i.e. the image area 21 stays fixed and the position of image stream IM1 within image area 21, that is the movie is displayed within image area 21 without vertical and/or lateral movement. Advantageously, according to this embodiment, the display device 14 comprises two image areas, namely the image area 21 displaying the image stream IM1 and the frame-like area 22 displaying the at least one second image IM2 which surrounds or frames the image stream IM1 and moves in accordance with the vehicle's acceleration. Regarding movement of the second image IM2, it is sufficient if a part of the image IM2 moves in accordance with the vehicle's acceleration, as long as the moving portion of the image IM2 can visually be captured by the user and is large enough such that visual information is no longer different from the passenger's body's sensory information received during vehicle motion to reduce the feeling of motion sickness.

For moving the second image IM2, or a part thereof, the processing device 13 determines at least one parameter which is indicative of at least one acceleration from the acceleration sensor data AD1, AD2, and/or AD3 during motion of the vehicle. The processing device 13 then sends control signals to the display device 14 which cause at least one portion of the frame-like area 22, in this embodiment the second image IM2 displayed within the frame-like area 22, to move during motion of the vehicle 1 according to the determined at least one parameter. For example, if the sensor data AD1 indicate a vertical acceleration of the vehicle in downward direction, e.g. when driving into a depression of the road, the second image IM2 is also moved vertically downward, preferably with the same or similar acceleration magnitude as sensed by the sensor device 15, and vertically upward when the vehicle leaves the depression of the road. As a result, the image IM2 vibrates in vertical direction VD in accordance with the vehicle 1 driving through a depression of the road. Similarly, the image IM2 is moved in lateral direction LD left and right or vice versa in accordance with the vehicle 1 driving through a curve of the road which causes the acceleration sensor device 16 to sense a lateral acceleration, which is then provided to the processing device 13.

According to an embodiment, the image stream IM1 (i.e., plurality of first images) and the at least one second image IM2 may be generated and displayed on the display device 14, e.g., by overlaying the image stream IM1 onto the second image IM2 so that the images IM1, IM2 are superimposed with each other, wherein the image stream IM1 occludes the second image IM2 except for the frame-like area 22. According to another embodiment, the second image IM2 may be generated as a frame-like image which surrounds the image stream IM1 or image area 21 with the frame-like area 22.

Thus, according to the invention, the frame-like area 22 (which is perceived by the user like a "virtual frame") shows a structure (as visible from the second image IM2) which is moving due to the measured acceleration. At the same time, only a small amount of the overall display area of the display device 14 may be used for the frame-like area 22, so that the display area 21 is not reduced significantly.

According to an embodiment, the second image IM2 may be a two-dimensional image, e.g. an infill image such as an image showing a certain pattern, which is moved in at least one of a vertical direction VD and lateral direction LD of the vehicle 1 corresponding to at least one of the sensed vertical acceleration AD1 and lateral acceleration AD2.

According to another embodiment, the at least one second image IM2 may be a three-dimensional image, which also depicts elements in longitudinal (e.g. perspective) view. With such image, which is moved by the processing device 13 in vertical direction VD, lateral direction LD, and/or longitudinal direction LOD of the vehicle 1 corresponding to at least one of the vertical acceleration, lateral acceleration, and longitudinal acceleration sensed by the sensor devices 15, 16, 17, motion sickness can be reduced also with respect to longitudinal acceleration (e.g. when the vehicle is sharply braking or accelerating).

Fig. 4B shows a schematic depiction of a display provided by a display device of a vehicle image displaying apparatus 10 according to another embodiment of the invention. In this embodiment, the display device 14 comprises at least one frame 30 comprising a frame-like area 22, similarly as in Fig. 4A. However, the frame-like area 22 can be any type of frame-like area, i.e. does not need to be a display area of the display screen. Particularly, the frame 30 is arranged around the display area 21 of the display device 14 displaying the image stream IM1 such that the image stream IM1 is framed by the frame-like area 22. In a preferred embodiment, the at least one frame 30 comprises one or more light emitting elements, such as light emitting diodes LEDs arranged in at least one portion of the frame-like area 22. When the vehicle is moving and subjected to various accelerations, the LEDs in the frame-like area 22 may move accordingly mirroring the same or similar accelerations, so that the moving LEDs can visually be captured by the user and the visual information is no longer different from the passenger's body's sensory information received during vehicle motion to reduce the feeling of motion sickness.

Regarding the control of the LEDs, the display device 14 may be equipped with corresponding actuators (not shown) for moving the LEDs in vertical direction VD, lateral direction LD, and/or longitudinal direction LOD, and which may be driven or controlled by the processing device 13 accordingly.

In both embodiments, it is preferable that the at least one parameter determined by the processing device 13 is indicative of at least one acceleration direction (such as vertical direction VD, and/or lateral direction LD) of the vehicle 1, and the at least one portion of the frame-like area 22, such as at least part of the image IM2 or the LEDs, is moved at least in one or more directions parallel to the at least one acceleration direction VD and/or LD. For example, if the acceleration is in vertical direction VD, the frame-like area 22 is moved parallel to the acceleration direction, i.e. vertically, preferably with same or proportional acceleration magnitude and same direction. Similarly, if the at least one parameter is indicative of at least one first acceleration magnitude of a sensed acceleration (such as vertical acceleration magnitude), the at least one portion of the frame-like area 22 is preferably moved with at least one second acceleration magnitude corresponding to the at least one first acceleration magnitude. For example, the second acceleration magnitude is the same as the first acceleration magnitude, or is directly proportional to the first acceleration magnitude.

According to an embodiment of the invention, the user may adjust, for example, a dimension, a movement sensitivity (e.g. a magnitude of the acceleration of the movement of the structure within the frame-like area 22), and/or a geometry of the frame-like area 22 through input in a human machine interface, such as a human machine interface 19 displayed on the display device 14 (Fig. 3).

## Claims

1. A method of displaying image data on a display device (14) disposed in a vehicle (1), comprising:
- receiving, by a processing device (13) comprising a processor (16) and adapted to be coupled with the display device (14), at least one first image (IM1) for displaying on the display device (14),
- displaying, by the processing device (13), the at least one first image (IM1) on the display device (14),
- providing a frame-like area (22) simultaneously with displaying the at least one first image (IM1) such that the at least one first image (IM1) is framed by the frame-like area (22) surrounding the at least one first image (IM1), wherein a position of at least a portion of the frame-like area (22) is controllably movable by the processing device (13),
- determining, by the processing device (13), at least one parameter which is indicative of at least one acceleration (AD1, AD2, AD3) of at least a part of the vehicle (1) during motion of the vehicle,
- moving, by the processing device (13), during motion of the vehicle (1) the at least one portion of the frame-like area (22) according to the at least one parameter.

2. The method according to claim, wherein the at least one parameter is determined from one or more sensor data (AD1, AD2, AD3) provided by at least one acceleration sensor device (15, 16, 17).

3. The method according to one of claims 1 to 2, wherein the at least one parameter is indicative of at least one acceleration direction (VD, LD) of the at least one part of the vehicle (1), and the at least one portion of the frame-like area (22) is moved at least in one or more directions parallel to the at least one acceleration direction (VD, LD).

4. The method according to one of claims 1 to 3, wherein the at least one parameter is indicative of at least one first acceleration magnitude of the at least one part of the vehicle (1), and the at least one portion of the frame-like area (22) is moved with at least one second acceleration magnitude corresponding to the at least one first acceleration magnitude.

5. The method according to one of claims 1 to 4, wherein the at least one parameter is indicative of at least one of a vertical acceleration (AD1) and a lateral acceleration (AD2) of the at least one part of the vehicle (1).

6. The method according to one of claims 1 to 4, wherein the at least one parameter is indicative of at least one of a vertical acceleration (AD1), a lateral acceleration (AD2), and a longitudinal acceleration (AD3) of the at least one part of the vehicle (1).

7. The method according to one of claims 1 to 6, comprising
- receiving, by the processing device (13), at least one second image (IM2) for displaying on the display device (14), wherein
- providing the frame-like area (22) comprises displaying the at least one second image (IM2) simultaneously with the at least one first image (IM1) on the display device (14) such that the at least one first image (IM1) is framed by the at least one second image (IM2) in the frame-like area (22), and moving the at least one portion of the frame-like area (22) comprises moving at least one portion of the at least one second image (IM2).

8. The method according to claim 7, wherein
- the at least one parameter is indicative of at least one of a vertical acceleration (AD1) and a lateral acceleration (AD2) of the at least one part of the vehicle (1), and
- the at least one second image (IM2) is a two-dimensional image which is moved in at least one of a vertical and lateral direction (VD, LD) of the vehicle (1) corresponding to at least one of the vertical acceleration (AD1) and lateral acceleration (AD2).

9. The method according to claim 7, wherein
- the at least one parameter is indicative of at least one of a vertical acceleration (AD1), a lateral acceleration (AD2), and a longitudinal acceleration (AD3) of the at least one part of the vehicle (1), and
- the at least one second image (IM2) is a three-dimensional image which is moved in at least one of a vertical (VD), a lateral (LD), and a longitudinal direction (LOD) of the vehicle (1) corresponding to at least one of the vertical acceleration (AD1), lateral acceleration (AD2), and longitudinal acceleration (AD3).

10. The method according to one of claims 1 to 9, wherein at least one of a dimension and a geometry of the frame-like area (22) is adjustable through input in a human machine interface (19) by a user.

11. A computer program product comprising software code sections which are adapted to perform a method according to one of the preceding claims when loaded into an internal memory of the processing device (13).

12. Vehicle image displaying apparatus (10), comprising:
- a display device (14) mounted in a vehicle (1) and configured to display one or more images,
- a processing device (13) comprising a processor (16) and adapted to be coupled with the display device (14), and configured to receive and display at least one first image (IM1) on the display device (14),
- at least one of the processing device (13) and the display device (14) is configured to provide a frame-like area (22) simultaneously with displaying the at least one first image (IM1) on the display device (14) such that the at least one first image (IM1) is framed by the frame-like area (22) surrounding the at least one first image (IM1), wherein a position of at least a portion of the frame-like area (22) is controllably movable by the processing device (13),
- the processing device (13) configured to determine at least one parameter which is indicative of at least one acceleration (AD1, AD2, AD3) of at least a part of the vehicle (1) during motion of the vehicle,
- the processing device (13) configured to move during motion of the vehicle (1) the at least one portion of the frame-like area (22) according to the at least one parameter.

13. The vehicle image displaying apparatus according to claim 12, wherein the processing device (13) is configured to display at least one second image (IM2) simultaneously with the at least one first image (IM1) on the display device (14) such that the at least one first image (IM1) is framed by the at least one second image (IM2) in the frame-like area (22), and the processing device is configured to move at least one portion of the at least one second image (IM2).

14. The vehicle image displaying apparatus according to claim 12, wherein the display device (14) comprises at least one frame (30) comprising the frame-like area (22), wherein the at least one frame (30) is arranged around a display area (21) of the display device (14) for displaying the at least one first image (IM1) such that the at least one first image (IM1) is framed by the frame-like area (22).

15. The vehicle image displaying apparatus according to claim 14, wherein the at least one frame (30) comprises one or more light emitting elements (LED) arranged in the at least one portion of the frame-like area (22).
